# EUROPEAN PATENT APPLICATION

(11) **EP 3 590 356 A1**
(43) Date of publication of application: **08.01.2020**
(21) Application number: 19382192.3
(22) Date of filing: 19.03.2019
(51) Int. Cl.: A23L 2/52, A23L 33/105, A23L 33/11, A61K 31/05, A61K 31/366, A61K 31/575, A61K 45/06, A61P 3/06, A61P 9/00

(54) **COMPOSITION FOR CONTROLLING AND REDUCING BLOOD CHOLESTEROL LEVELS**

(30) Priority: 05.07.2018 EP 18382501; 01.08.2018 EP 18382584
(71) Applicant: Uriach Consumer Healthcare, S.L., 08184 PALAU-SOLITÀ I PLEGAMANS (ES)
(72) Inventor: Raventós Colomer, Rosa Maria, 08184 PALAU-SOLITÀ I PLEGAMANS (ES); Fortuny Solà, Ana, 08184 PALAU-SOLITÀ I PLEGAMANS (ES); Sala Llinares, Alberto, 08757 CORBERA DE LLOBREGAT (ES); Fernández Navarro, Carlos, 08907 HOSPITALET DE LLOBREGAT (ES)
(74) Representative: ZBM Patents - Zea, Barlocci & Markvardsen

(57) **Abstract**

The present invention relates to a composition comprising a) phytosterols and/or phytostanols, or an extract comprising phytosterols and/or phytostanols; b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof; and c) hydroxytyrosol, or an extract comprising Hydroxytyrosol, Capable of controlling and reducing blood cholesterol levels in an individual.

## Description

### Technological field

The invention relates to a composition capable of controlling and reducing blood cholesterol levels. In turn, the invention also relates to the preparation of said composition and the use thereof, specifically, for controlling and reducing blood cholesterol levels of patients with hypercholesterolemia.

### State of the Art

Hypercholesterolemia affects one out of every two adults in Spain and is one of the main risk factors of arteriosclerotic vascular disease. Its major atherothrombotic complications generate high morbidity and are the leading cause of death worldwide. Therefore, the prevention and treatment of hypercholesterolemia in the context of the comprehensive cardiovascular risk management is a crucial aspect for doctors and healthcare professionals, such as pharmacists and nutritionists. Despite solid scientific evidence which supports the cardiovascular benefit of lipid-lowering drugs such as statins and/or ezetimibe, dietary supplements which aim to reduce cholesterol plasma numbers and improve the lipid profile may be of great help in both preventing and initially managing patients with a vascular risk and even patients with established cardiovascular disease.

Monacolin K, which comes from Monascus purpureus or red yeast rice, inhibits 3-hydroxy-methyl-glutaryl (HMG)-CoA reductase, a limiting enzyme in endogenous cholesterol biosynthesis, and according to the European Food Safety Authority (EFSA), contributes to maintaining normal blood cholesterol levels, with its effect mostly targeting the control of endogenous cholesterol.

Other agents capable of treating dyslipidemia are, for example, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine or pantothenic acid.

The EFSA Panel, in turn, considers that hydroxytyrosol and its derivatives (e.g. oleuropein and tyrosol) in olive oil help to maintain a normal HDL-cholesterol plasma concentration and protects LDL-cholesterol particles against oxidation.

In turn, the capability of phytosterols, plant sterols, of reducing LDL cholesterol levels, has also been demonstrated. Likewise, a recent metanalysis of 11 randomised clinical trials which included 1970 different nutraceutical combinations administered to 3924 subjects, confirmed the benefit of these compounds in improving the lipid profile. Phytosterols positively influence the control of blood cholesterol relating to its intestinal absorption, i.e. they help in the control of cholesterol levels on an exogenous level.

The origin of hypercholesterolemia may be different according to whether it is due to a defect in the absorption of cholesterol from the diet, an excessive intake of foods containing it, or whether the dysfunction is related to the internal metabolism of cholesterol. Based on what is known in the state of the art, it can be seen that there is still a need to find combinations useful in the prevention and treatment of hypercholesterolemia which can be highly effective regardless of the origin of said condition.

### Summary of the Invention

The inventors have found that the combination according to the invention provides an improved effect on the control and reduction of cholesterol levels. The combination of at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof, hydroxytyrosol and phytosterols results in a highly effective restoration of cholesterol levels because synergistic effect takes place between the mentioned active ingredients. In particular, if phytosterols, Monacolin K and hydroxytyrosol are combined, and even more particularly, in given proportions, the effect may be even more pronounced.

Thus, a composition comprising particularly Phytosterols and/or phytostanols, or an extract comprising phytosterols and/or phytostanols; Monacolin K, or an extract comprising Monacolin K; and Hydroxytyrosol, or an extract comprising Hydroxytyrosol, is highly effective in restoring cholesterol levels of patients to levels within medical standards, in addition to providing a reduction of the inflammatory component present in patients of this type.

Furthermore, the composition of the invention can be prepared by a simple method that is easy to industrialise, and it furthermore has the advantage of enabling administration by oral route in numerous formats which facilitates the acceptance and dosage in relation to the patient.

Accordingly, one aspect of the present invention relates to a composition comprising:
a) phytosterols and/or phytostanols, or an extract comprising phytosterols and/or phytostanols;
b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof; and
c) hydroxytyrosol, or an extract comprising hydroxytyrosol.

Preferably, the agent capable of treating dyslipidemia is selected from Monacolin K or an extract comprising Monacolin K.

Optionally, the composition may comprise other components, such as pharmaceutically and/or food-grade acceptable excipients and/or other food supplements, for example. In a preferred embodiment of the present invention, the other components are selected from vitamin E, berberine, for example obtained from a *Berberis vulgaris* extract, lycopene, coenzyme Q10, Ubiquinol, artichoke, for example obtained from a *Cynara scolymus* extract, desmodium, for example obtained from a *Desmodium adscendens* extract, black radish, for example obtained from a *Raphanus sativus nigra* extract, milk thistle, for example obtained from a *Silybum marianum* extract, policosanols, beta-glucans from barley or oat, and/or carob bean; at least one filler, at least one thickener, at least one preservative, at least one acidulant, at least one flavouring and mixtures thereof, optionally as well as a liquid vehicle until reaching 100 % by weight of the composition.

Another aspect of the present invention relates to a method for preparing the composition, which method comprises mixing the following components:
a) phytosterols and/or phytostanols, or an extract comprising phytosterols and phytostanols;
b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof;
c) Hydroxytyrosol, or an extract comprising Hydroxytyrosol; and
d) optionally, at least one ingredient selected from the group consisting of: vitamin E, berberine (Berberis vulgaris extract), lycopene, coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean; and
e) optionally one or more ingredients identical to or different from one another selected from: a filler, a thickener, a preservative, a stabiliser, an acidulant, a sweetener and a flavouring; and
f) optionally, a liquid vehicle until reaching 100 % by weight of the composition.

Another aspect of the invention relates to a food comprising the composition as defined in the first aspect, together with one or more edible excipients or vehicles. These foods are referred to as functional foods or dietary supplements.

Another aspect of the present invention relates to a pharmaceutical composition comprising a therapeutically effective amount of the composition as defined in the first aspect, together with pharmaceutically acceptable excipients or carriers.

Lastly, another aspect of the present invention relates to a composition as defined in the first aspect, for use in controlling and reducing blood cholesterol levels of an individual.

### Detailed Description of the Invention

Unless indicated otherwise, all the expressions such as those used in this application, must be understood according to their common meaning as they are known in the art. Other more specific definitions for certain expressions such as those used in the present application are explained below, and they are intended to be applied uniformly throughout the specification and claims unless a definition expressly stating otherwise provides a broader definition.

As discussed above, the composition of the invention has a high capability of modulating blood cholesterol levels and acts effectively for these levels to lower and even return to baseline levels, thereby reducing the risk of said patients developing pathologies derived from hypercholesterolemia such as arteriosclerosis. Therefore, one aspect of the invention relates to a composition comprising: a) phytosterols and/or phytostanols, or an extract comprising phytosterols and/or phytostanols; b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium, Hydrastis canadensis, Xanthorhiza simplicissima, Phellodendron amurense, Coptis chinensis, Tinospora cordifolia, Argemone mexicana and Eschscholzia californica), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof; and c) hydroxytyrosol, or an extract comprising hydroxytyrosol.

In particular, it has been observed that the preferred composition in which the agent capable of treating dyslipidemia is Monacolin K or an extract comprising Monacolin K according to the invention reduces cholesterol levels in patients by 15 % in one month. Therefore, a preferred aspect of the invention is a composition comprising: a) phytosterols and/or phytostanols, or an extract comprising phytosterols and/or phytostanols; b) Monacolin K, or an extract comprising Monacolin K; and c) hydroxytyrosol, or an extract comprising hydroxytyrosol.

The mixture of phytosterols and/or phytostanols, or the extract comprising phytosterols and/or phytostanols; the Monacolin K, or the extract comprising Monacolin K; and the hydroxytyrosol, or the extract comprising hydroxytyrosol, are found in the composition of the invention in an amount such that it produces a synergistic effect between them in the control or reduction of cholesterol levels.

The phytosterols or plant sterols (sterols from plants) are natural sterols of plant origin. Thus, the term "phytosterol" or "phytosterols" used in the description of the present invention refers to plant sterols. Examples of phytosterols are the sitosterol, in particular beta-sitosterol, campsterol, stigmasterol, brassicasterol and ergosterol, among others. "Phytostanols" are saturated plant sterols. Examples of phytostanols are sitostanol, in particular the beta-sitostanol and campstanol, among others. In one embodiment of the invention, the extract comprising phytosterols comprises beta-sitosterol, campsterol, stigmasterol, and brassicasterol. In another embodiment of the invention, the extract comprising phytostanols comprises beta-sitostanol and campstanol. In yet another embodiment of the invention, the extract comprising a mixture of phytosterols and phytostanols comprises beta-sitosterol, campsterol, stigmasterol, brassicasterol, beta-sistostanol and campstanol. In a preferred embodiment, the extract comprises a mixture of phytosterols and phytostanols.

All the % by weight (% weight/weight) referred to throughout the description are expressed in relation to the total weight of the composition, unless indicated otherwise.

The given ranges, such as temperatures, times, sizes and the like, must be considered approximate, unless specifically indicated otherwise. Likewise, any given range includes both the lower end point and the upper end point of the range.

In another embodiment of the invention, optionally combined with any other embodiment of the invention described herein above or below, the beta-sitosterol content in the mixture of phytosterols and phytostanols is equal to or less than 80 % by weight. In another embodiment of the invention, optionally combined with any other embodiment of the invention described herein above or below, the campsterol content in the mixture of phytosterols and phytostanols is equal to or less than 40 % by weight. In another embodiment of the invention, optionally combined with any other embodiment of the invention described herein above or below, the content of stigmasterol in the mixture of phytosterols and phytostanols is equal to or less than 30 % by weight. In another embodiment of the invention, optionally combined with any other embodiment of the invention described herein above or below, the brassicasterol content in the mixture of phytosterols and phytostanols is equal to or less than 3 % by weight. In another embodiment of the invention, optionally combined with any other embodiment of the invention described herein above or below, the beta-sitostanol content in the mixture of phytosterols and phytostanols is equal to or less than 15 % by weight. In another embodiment of the invention, optionally combined with any other embodiment of the invention described herein above or below, the content of campstanol in the mixture of phytosterols and phytostanols is equal to or less than 5 % by weight.

In another embodiment of the invention, optionally combined with any other embodiment of the invention described herein above or below, the % by weight of beta-sitosterol is equal to or less than 80, the % by weight of campsterol is equal to or less than 40, the % by weight of stigmasterol is equal to or less than 30, the % by weight of brassicasterol is equal to or less than 3, the % by weight of beta-sitostanol is equal to or less than 15, and the % by weight of campstanol is equal to or less than 5 % with respect to the total weight of the mixture of phytosterols and phytostanols; where the sum of beta-sitosterol, campsterol, stigmasterol, brassicasterol, betasitostanol and campstanol gives 100 % by weight.

The phytosterols can be isolated from natural sources. Such natural sources are usually vegetable oils, such as soybean oil, rapeseed (canola) oil, sunflower or corn oil, or the so-called "tall oil", a by-product of the production of wood pulp. These sterols can be hydrogenated to obtain phytostanols. Both the phytosterols and the phytostanols, which are powders with a high melting point (130 °C-170 °C), can be esterified with fatty acids of plant origin. The resulting esters are liquid or semi-liquid materials which have chemical and physical properties that are comparable to edible fats and oils, which allows supplementing numerous processed foods with phytosterol and phytostanol esters.

Processes for obtaining phytosterols and/or phytostanols are well known to one skilled in the art. For example, in the case of being obtained from vegetable oils, an extraction is carried out using oilseeds, and then a refining process typically follows to remove minor components from the oil such as phosphatides, free fatty acids, pigments and odours, with the least possible damage to glycerides and minimum oil loss.

In one embodiment of the invention, optionally combined with any other embodiment of the invention described herein above or below, the % by weight of the mixture of phytosterols and/or phytostanols in the phytosterol and/or phytostanol extract is comprised between 1 % and 100 %, preferably between 50 % and 100 %, and even more preferably between 75 % and 100 %. In another embodiment, optionally combined with any other embodiment of the invention described herein above or below, the % by weight of the mixture of phytosterols and phytostanols in the phytosterol and phytostanol extract is comprised between 85 % and 99 %. In yet another embodiment, optionally combined with any other embodiment of the invention described herein above or below, the % by weight of the mixture of phytosterols and phytostanols in the phytosterol and phytostanol extract is comprised between 90 % and 99 %. In a preferred embodiment, optionally combined with any other embodiment of the invention described herein above or below, the % by weight of the mixture of phytosterols and phytostanols in the phytosterol and phytostanol extract is comprised between 95 % and 99 %.

In another embodiment of the invention, optionally combined with any other embodiment of the invention described herein above or below, the mixture of phytosterols and phytostanols as mentioned above has a % by weight in the phytosterol and phytostanol extract selected from the following amounts: 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 %.

Monacolins are a group of molecules produced by fermentation of rice by the yeast *Monascus purpureus.* Fermentation of the species *Monascus purpureus* has been known for centuries in plant therapy and nutrition in China. The yeast is added to the rice (Oryza sativa) to allow its fermentation and the obtained product, known as red rice, is used in the food sector.

Monacolins have a structure similar to that of statins. Monacolin K (formula I) has the same structure as lovastatin, as shown below:

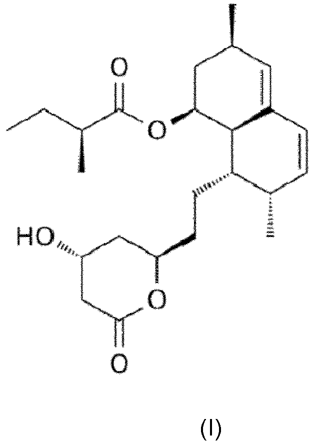

The terms "red yeast rice", "red rice yeast", "red rice", "product of red rice", "extract of red rice" and the like refer to a product resulting from the fermentation performed by at least one fungus of the species *Monascus.* This fermentation can also be carried out by a mixture of fungi of the species *Monascus.*

Fermented red rice can be obtained through known methods, for example as described in European patent EP 1044009 B1 (Detailed description of the invention; Preparation of conventional culture fluid, pages 5 to 6), the teaching of which is incorporated herein by reference. To obtain the red rice, one or more natural or mutating strains of fungi of the species *Monascus* can be used, such as those comprised in the following list: *Monascus ruber van Tieghem, Monascus Paxii Lingelsheim, Monascus purpureus, Monascus Fuliginosus* and *Monascus albidus.*

In one embodiment of the invention, optionally combined with any other embodiment of the invention described herein above or below, the red yeast rice extract of the present invention contains an amount of Monacolin K comprised between 0.001 % and 100 %, preferably between 0,001 % and 50 %, more preferably between 0.001 % and 25 %, and even more preferably between 0.001 % and 15 % by weight of the extract. In another embodiment, the red yeast rice extract of the present invention contains an amount of Monacolin K comprised between 0.01 % and 10 % by weight of the extract. In yet another embodiment, the red yeast rice extract of the present invention contains an amount of Monacolin K comprised between 0.05 % and 10 % by weight of the extract.

In another embodiment of the invention, optionally combined with any other embodiment of the invention described herein above or below, the red yeast rice extract as mentioned above comprises an amount of Monacolin K selected from the following amounts: 0,001 %, 0,005 %, 0.01 %, 0.05 %, 0.1 %, 0.5 %, 1 %, 2 %, 3 %, 4 %, 5 %, 6 %, 7 %, 8 %, 9 %, 10 %, 11 %, 12 %, 13 %, 14 %, 15 %.

Hydroxytyrosol (3,4-dihydroxyphenylethanol; DOPET) is a phytochemical polyphenol with antioxidant properties. Its coefficient of oxygen radical absorbance capacity (ORAC) is 40,000 µmolTE/g.

In nature, hydroxytyrosol is present in olive trees, *Olea europaea,* mostly concentrated in the leaves, where it acts as an immunostimulant and antibiotic. Similarly, it is present in extra virgin olive oil (and not in the other oils, since it is removed by refining), both in its free form and in the form of oleuropein, which are responsible for the bitter taste.

Hydroxytyrosol can be obtained by chemical or enzymatic synthesis and by extraction and purification from oil industry by-products.

Specifically, it can be obtained by mild hydrolysis of the olive tree leaves, as described in the paper by De la Fuente et al. (De la Fuente P, Chamorro P, Moreno M., Poza M.A., "Propiedades antioxidantes del hidroxitirosol procedente de la hoja de olivo (Olea europea L.)", Revista de Fitoterapia 2004, 4, 139-147), or by hydrolysis of its esterified form, oleuropein, which was previously obtained, by different methods as described in papers such as those written by Fleming et al. (Fleming H P, Walter W M, Etchells J L, "Antimicrobial properties of Oleuropein and products of its hydrolysis from green olives" App. Microbio 1973; 26: 777-782) and Yuan et al. (Yuan JJ, Wang CZ, Ye JZ, Tao R, Zhang YS, "Enzymatic hydrolysis of oleuropein from Olea eropea (olive) leaf extract and antioxidant activities" Molecules 2015, 20(2):2903-21).

In turn, oleuropein can be extracted from olives using the method described in European patent EP 1098573 B1 (see pages 3 and 4) or from olive tree leaves using the method described in patent US 5714150 (see page 2), the teachings of which are incorporated herein by reference.

In one embodiment of the invention, optionally combined with any other embodiment of the invention described herein above or below, the extract comprising hydroxytyrosol of the present invention contains an amount of hydroxytyrosol comprised between 1 % and 100 %, preferably between 1 % and 75 %, more preferably between 3 % and 50 % and even more preferably between 5 % and 40 % by weight of the extract. In another embodiment, the extract comprising hydroxytyrosol of the present invention contains an amount of hydroxytyrosol comprised between 10 % and 35 % by weight of the extract. In yet another embodiment, the extract comprising hydroxytyrosol of the present invention contains an amount of hydroxytyrosol comprised between 15 % and 30 % by weight of the extract. In a preferred embodiment, the extract comprising hydroxytyrosol of the present invention contains an amount of hydroxytyrosol comprised between 15 % and 25 % by weight of the extract.

In another embodiment of the invention, optionally combined with any other embodiment of the invention described herein above or below, the extract comprising hydroxytyrosol as mentioned above comprises an amount of hydroxytyrosol selected from the following amounts: 5 %, 6 %, 7 %, 8 %, 9 %, 10 %, 11 %, 12 %, 13 %, 14 %, 15 %, 16 %, 17 %, 18 %, 19 %, 20 %, 21 %, 22 %, 23 %, 24 %, 25 %, 26 %, 27 %, 28 %, 29 %, 30 %, 31 %, 32 %, 33 %, 34 %, 35 %, 36 %, 37 %, 38 %, 39 % and 40 %.

In another embodiment of the invention, optionally combined with any other embodiment of the invention described herein above or below, the composition as defined in the first aspect, comprises phytosterols and/or phytostanols between 1 and 25 % by weight with respect to the composition, preferably between 5 and 15 %, more preferably between 7 and 14 %, and even more preferably between 9 and 13 %. In particular, the % by weight with respect to the composition of phytosterols and/or phytostanols can be any value of 9 %, 9.5 %, 10 %, 10.5 %, 11 %, 11.5 %, 12 %, 12.5 % and 13 %, and any combination of the mentioned values forming a range. The composition further comprises Monacolin K between 0.01 and 0.2 % by weight with respect to the composition, preferably between 0.03 and 0.15 %, more preferably between 0.05 and 0.12 %, and even more preferably between 0.06 and 0.09 %. In particular, the % by weight with respect to the composition of Monacolin K can be any value of 0.06 %, 0.065 %, 0.07 %, 0.075 %, 0.08 %, 0.085 % and 0.09 %, and any combination of the mentioned values forming a range. The composition further comprises Hydroxytyrosol between 0.005 and 2 % by weight with respect to the composition, preferably between 0.01 and 0.08 %, more preferably between 0,015 and 0,065 %, and even more preferably between 0.02 and 0.05 %. In particular, the % by weight with respect to the composition of Hydroxytyrosol can be any value of 0.02 %, 0.025 %, 0.03 %, 0.035 %, 0.04 %, 0.045 % and 0.05 %, and any combination of the mentioned values forming a range.

In another embodiment of the invention, optionally combined with any other embodiment of the invention described herein above or below, the composition as defined in the first aspect, comprises an extract comprising phytosterols and/or phytostanols between 5 and 30 % by weight with respect to the composition, preferably between 5 and 20 %, more preferably between 7.5 and 15 %, and even more preferably between 9 and 13 %. In particular, the % by weight with respect to the composition of the extract comprising phytosterols and/or phytostanols can be any value of 9 %, 9.5 %, 10 %, 10.5 %, 11 %, 11.5 %, 12 %, 12.5 % and 13 %, and any combination of the mentioned values forming a range. The composition further comprises an extract comprising Monacolin K between 0.5 and 5 % by weight with respect to the composition, preferably between 0.75 and 4 %, more preferably between 1 and 3 %. In particular, the % by weight with respect to the composition of Monacolin K can be any value of 1 %, 1.5 %, 2 %, 2.5 %, 3 %, 3.5 %, 4 %, 4.5 % and 5 %, and any combination of the mentioned values forming a range. The composition further comprises an extract comprising Hydroxytyrosol between 0.02 and 8 % by weight with respect to the composition, preferably between 0.02 and 4 %, more preferably between 0.05 and 1 %, more preferably between 0,075 and 0.8 %, and even more preferably between 0.1 and 0.6 %. In particular, the % by weight with respect to the composition of the extract comprising Hydroxytyrosol can be any value of 0.1 %, 0.15 %, 0.2 %, 0.25 %, 0.3 %, 0.35 %, 0.4 %, 0.45 %, 0.5 %, 0.55 % and 0.6 %, and any combination of the mentioned values forming a range.

In one embodiment of the invention, optionally combined with any other embodiment of the invention described herein above or below, the composition as defined in the first aspect, comprises phytosterols and/or phytostanols in an amount for daily administration of between 0.1 g and 10 g, preferably between 0.5 g and 7 g, more preferably between 1 g and 5 g, even more preferably between 1.5 g and 5 g, even more preferably between 1.5 g and 3.5 g. In particular, the amount for daily administration of phytosterols and/or phytostanols can be any value of 0.5 g, 0.6 g, 0.7 g, 0.8 g, 0.9 g, 1.0 g, 1.1 g, 1.2 g, 1.3 g, 1.4 g, 1.5 g, 1.6 g, 1.7 g, 1.8 g, 1.9 g, 2 g, 2.1 g, 2.2 g, 2.3 g, 2.4 g, 2.5 g, 2.6 g, 2.7 g, 2.8 g, 2.9 g, 3.0 g, 3.1 g, 3.2 g, 3.3 g, 3.4 g, and 3.5 g, and any combination of the mentioned values forming a range. The composition further comprises Monacolin K in an amount for daily administration of between 0.1 mg and 20 mg, preferably between 0.5 mg and 15 mg, more preferably between 1 mg and 12 mg, even more preferably between 3 mg and 12 mg. In particular, the amount for daily administration of Monacolin K can be any value of 1 mg, 2 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, and 12 mg, and any combination of the mentioned values forming a range. The composition further comprises Hydroxytyrosol in an amount for daily administration of between 0.1 mg and 20 mg, preferably between 0.5 mg and 15 mg, more preferably between 1 mg and 12 mg, even more preferably between 3 mg and 12 mg. In particular, the amount for daily administration of Hydroxytyrosol can be any value of 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, and 12 mg, and any combination of the mentioned values forming a range.

These amounts refer to the amount of active ingredient with respect to the total composition.

In one embodiment of the invention, optionally combined with any other embodiment of the invention described herein above or below, the composition as defined in the first aspect, comprises an extract comprising phytosterols and/or phytostanols in an amount for daily administration of between 0.1 g and 13.3 g, preferably between 0.5 g and 9.5 g, more preferably between 1 g and 7 g, even more preferably between 1.5 g and 6.5 g, even more preferably between 1.5 g and 4.5 g. In particular, the amount for daily administration of the extract comprising phytosterols and/or phytostanols can be any value of 1.5 g, 1.6 g, 1.7 g, 1.8 g, 1.9 g, 2 g, 2.1 g, 2.2 g, 2.3 g, 2.4 g, 2.5 g, 2.6 g, 2.7 g, 2.8 g, 2.9 g, 3.0 g, 3.1 g, 3.2 g, 3.3 g, 3.4 g, 3.5 g, 3.6, 3.7, 3.8, 3.9, 4.0, 4.1, 4.2, 4.3, 4.4 and 4.5, and any combination of the mentioned values forming a range. The composition further comprises an extract comprising Monacolin K in an amount for daily administration of between 0.7 mg and 1 g, preferably between 3.5 mg and 500 mg, more preferably between 7 mg and 250 mg, even more preferably between 20 mg and 250 mg. In particular, the amount for daily administration of extract comprising Monacolin K can be any value of 20 mg, 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg and 250 mg, and any combination of the mentioned values forming a range. The composition further comprises an extract comprising Hydroxytyrosol in an amount for daily administration of between 0.25 mg and 400 mg, preferably between 1.25 mg and 300 mg, more preferably between 7.5 mg and 200 mg, even more preferably between 15 mg and 60 mg. In particular, the amount for daily administration of extract comprising Hydroxytyrosol can be any value of 15 mg, 20 mg, 25 mg, 30 mg, 40 mg, 45 mg, 50 mg, 55 mg and 60 mg, and any combination of the mentioned values forming a range.

These amounts refer to the amount of extract comprising the active ingredient with respect to the total composition.

In another embodiment, optionally combined with any other embodiment of the invention described herein above or below, the ratio by weight between the amount of phytosterols and/or phytostanols/Monacolin K comprised in the composition is between 1:120 and 1:180, more specifically, between 1:130 and 1:160. In another embodiment, said ratio is 1:150.

In another embodiment, optionally combined with any other embodiment of the invention described herein above or below, the ratio by weight between the amount of Monacolin K/hydroxytyrosol comprised in the composition is between 1:1 and 1:5, more specifically, between 1:1 and 1:3. In another embodiment, said ratio is 1:2.

The inventors have observed that the addition of other antioxidant elements such as vitamin E, can positively influence the effect of the composition of the invention.

Therefore, in one embodiment of the invention, optionally combined with any other embodiment of the invention described herein above or below, the composition may optionally comprise another antioxidant element, more specifically, the composition further comprises vitamin E.

α-tocopherol or vitamin E is a liposoluble vitamin that acts as an antioxidant at the level of the synthesis of heme pigment, which is an essential part of red blood cell haemoglobin. There are numerous sources of vitamin E, most of which are of plant origin.

In another embodiment of the invention, optionally combined with any other embodiment of the invention described herein above or below, the % by weight of Vitamin E with respect to the weight of the composition is comprised between 0.01 and 5 %. In another embodiment, it is comprised between 0.05 and 1 %. In another embodiment, it is comprised between 0.1 and 0.5 %. In another embodiment, the % by weight of Vitamin E with respect to the composition is about 0.2 %.

In another embodiment of the invention, optionally combined with any other embodiment of the invention described herein above or below, the amount for daily administration of Vitamin E comprised in the composition can be between 1 and 1000 mg, preferably between 1 and 500 mg, more preferably between 1 and 250 mg, and even more preferably between 1 and 100 mg. In another embodiment, the vitamin E is comprised in a daily dose amount of between 1 and 50 mg, preferably between 2 and 40 mg, more preferably between 3 and 30 mg. In particular, the amount of vitamin E can be any value of 1 mg, 2 mg, 3 mg, 4 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, and 40 mg, and any combination of the mentioned values forming a range.

Other optional ingredients which can be added to the composition are berberine, for example obtained from a *Berberis vulgaris* extract), lycopene, coenzyme Q10, Ubiquinol, artichoke, for example obtained from a *Cynara scolymus* extract, desmodium (*Desmodium adscendens* extract), black radish (*Raphanus sativus nigra* extract), milk thistle (*Silybum marianum* extract), policosanols, beta-glucans (for example from barley or oat), and carob bean.

In one embodiment of the invention, optionally combined with any other embodiment of the invention described herein above or below, the composition further comprises at least one ingredient selected from the group consisting of: vitamin E, berberine (Berberis vulgaris extract), lycopene, coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean.

The composition may also contain other excipients such as: fillers, thickeners, preservatives, stabilisers, acidulants, flavourings, sweeteners and combinations thereof.

Fillers are substances which increase the volume of a food without contributing to its possible energy value. Examples of fillers are, without seeking to be limited to that described, microcrystalline cellulose, carboxyalkyl cellulose derivatives such as carboxymethyl cellulose sodium, combinations of microcrystalline cellulose and carboxymethyl cellulose, Xanthan, erythritol and polydextrose, among others.

Thickeners are substances which increase the viscosity of a food. Examples of thickeners are, without seeking to be limited to that described, glycerin, cellulose, methyl cellulose, agar-agar, pectin, methyl-ether-cellulose, among others.

Stabilisers are substances that prevent the separation of emulsions, foams or suspensions into their individual components upon increasing the viscosity of the mixture. Examples of stabilisers are, without seeking to be limited to that described, tapioca maltodextrin, corn maltodextrin and modified corn starch (A1450), among others.

Preservatives are substances which prolong the shelf life of food products by protecting them against deterioration caused by the microorganisms. Examples of preservatives are, without seeking to be limited to that described, potassium sorbate, sodium sorbate, calcium sorbate, potassium benzoate, sodium benzoate, calcium benzoate, potassium sulfite, sodium sulfite, calcium sulfite, among others.

Acidulants are substances which increase the acidity of a food or give it an acidic taste. Examples of acidulants are, without seeking to be limited to that described, citric acid, malic acid, ascorbic acid, acid anhydride, lactic acid and tartaric acid, among others.

Flavourings are those substances which provide taste to foods, modifying their organoleptic characteristics and making them sweeter, more bitter, saltier or more acidic. Examples of flavourings are, without seeking to be limited to that described, strawberry flavour, raspberry flavour, banana flavour, vanilla flavour, orange flavour, pear flavour and pineapple flavour, among others.

Sweeteners are those substances which provide a sweeter taste to foods. Examples of sweeteners are, without seeking to be limited to that described, stevia, sucralose, sorbitol and mannitol, among others.

Other ingredients can be ingredients such as sodium chloride and/or natural flavours to mask bitter tastes, also called anti-bitter natural flavours. For example, one such aroma is the one known by the name Tastegem, which contains flavouring components, tapioca maltodextrin, corn maltodextrin and modified corn starch (E 1450).

In another embodiment of the invention, optionally combined with any other embodiment of the invention described herein above or below, the composition of the invention may comprise one or more ingredients identical to or different from one another selected from: a filler, a thickener, a preservative, a stabiliser, an acidulant, a sweetener and a flavouring.

The decision to add one or more elements from the group mentioned in the preceding embodiment and/or the combination of some of them and in what proportion is part of the know-how of one skilled in the art.

In one embodiment, optionally combined with any other embodiment of the invention described herein above or below, the composition of the present invention is in solid, semi-solid, gel, suspension or liquid form.

In another embodiment, optionally combined with any other embodiment of the invention described herein above or below, the composition of the invention is in liquid form. Preferably, the composition in liquid form comprises a liquid vehicle.

Generally, the liquid vehicle of the composition is selected from pharmaceutically acceptable liquids, for example water, vegetable oil, milk, or ethanol. In one embodiment of the invention, optionally combined with any other embodiment of the invention described herein above or below, the composition is in liquid form, where the vehicle is water.

In one embodiment of the invention, optionally combined with any other embodiment of the invention described herein above or below, the composition of the invention comprises the mixture of
a) phytosterols and/or phytostanols, or an extract comprising phytosterols and/or phytostanols;
b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof;
c) Hydroxytyrosol, or an extract comprising Hydroxytyrosol; and
d) optionally, at least one ingredient selected from the group consisting of: vitamin E, berberine (Berberis vulgaris extract), lycopene, Coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean; and
e) optionally one or more ingredients identical to or different from one another selected from: a filler, a thickener, a preservative, a stabiliser, an acidulant, a sweetener and a flavouring; and f) optionally, water until reaching 100 % by weight of the composition.

In another embodiment of the invention, optionally combined with any other embodiment of the invention described herein above or below, the composition of the invention comprises the mixture of
a) phytosterols and/or phytostanols, or an extract comprising phytosterols and/or phytostanols;
b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof;
c) Hydroxytyrosol, or an extract comprising Hydroxytyrosol; and
d) at least one ingredient selected from the group consisting of: vitamin E, Berberine (Berberis vulgaris extract), lycopene, Coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean; and
e) optionally one or more ingredients identical to or different from one another selected from: a filler, a thickener, a preservative, a stabiliser, an acidulant, a sweetener and a flavouring; and
f) optionally, water until reaching 100 % by weight of the composition.

In another embodiment of the invention, optionally combined with any other embodiment of the invention described herein above or below, the composition of the invention comprises the mixture of
a) phytosterols and/or phytostanols, or an extract comprising phytosterols and/or phytostanols;
b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof;
c) Hydroxytyrosol, or an extract comprising Hydroxytyrosol; and
d) at least one ingredient selected from the group consisting of: vitamin E, Berberine (Berberis vulgaris extract), lycopene, Coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean; and
e) one or more ingredients identical to or different from one another selected from: a filler, a thickener, a preservative, a stabiliser, an acidulant, a sweetener and a flavouring; and
f) optionally, water until reaching 100 % by weight of the composition.

In another embodiment of the invention, optionally combined with any other embodiment of the invention described herein above or below, the composition of the invention comprises the mixture of
a) phytosterols and/or phytostanols, or an extract comprising phytosterols and/or phytostanols;
b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof;
c) Hydroxytyrosol, or an extract comprising Hydroxytyrosol; and
d) at least one ingredient selected from the group consisting of: vitamin E, Berberine (Berberis vulgaris extract), lycopene, Coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean; and
e) one or more ingredients identical to or different from one another selected from: a filler, a thickener, a preservative, a stabiliser, an acidulant, a sweetener and a flavouring; and
f) water until reaching 100 % by weight of the composition.

In another embodiment of the invention, optionally combined with any other embodiment of the invention described herein above or below, the composition of the invention consists of the mixture of
a) phytosterols and/or phytostanols, or an extract comprising phytosterols and/or phytostanols;
b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof;
c) Hydroxytyrosol, or an extract comprising Hydroxytyrosol; and
d) at least one ingredient selected from the group consisting of: vitamin E, Berberine (Berberis vulgaris extract), lycopene, Coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean; and
e) one or more ingredients identical to or different from one another selected from: a filler, a thickener, a preservative, a stabiliser, an acidulant, a sweetener and a flavouring; and
f) water until reaching 100 % by weight of the composition.

In a preferred embodiment of the invention, optionally combined with any other embodiment of the invention described herein above or below, the at least one agent capable of treating dyslipidemia in the composition of the invention is selected from Monacolin K, or an extract comprising Monacolin K.

The examples of the present invention describe in detail a method of preparation for a composition of the invention. As discussed above, an aspect of the invention is a method for preparing the composition of the invention comprising mixing the following components
a) phytosterols and/or phytostanols, or an extract comprising phytosterols and/or phytostanols;
b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof;
c) Hydroxytyrosol, or an extract comprising Hydroxytyrosol; and
d) optionally, at least one ingredient selected from the group consisting of: vitamin E, Berberine (Berberis vulgaris extract), lycopene, Coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean; and
e) optionally, one or more ingredients identical to or different from one another selected from: a filler, a thickener, a preservative, a stabiliser, an acidulant, a sweetener and a flavouring; and
f) optionally, water until reaching 100 % by weight of the composition.

In one embodiment of this aspect of the invention, optionally combined with any other embodiment of the invention described herein above or below, the method comprises the mixture of a) phytosterols and/or phytostanols, or an extract comprising phytosterols and/or phytostanols; b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof; c) Hydroxytyrosol, or an extract comprising Hydroxytyrosol; and d) at least one ingredient selected from the group consisting of: vitamin E, Berberine (Berberis vulgaris extract), lycopene, Coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean; and
e) optionally, one or more ingredients identical to or different from one another selected from: a filler, a thickener, a preservative, a stabiliser, an acidulant, a sweetener and a flavouring; and
f) optionally, water until reaching 100 % by weight of the composition.

In one embodiment of this aspect of the invention, optionally combined with any other embodiment of the invention described herein above or below, the method comprises the mixture of a) phytosterols and/or phytostanols, or an extract comprising phytosterols and/or phytostanols; b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof; c) Hydroxytyrosol, or an extract comprising Hydroxytyrosol; and d) at least one ingredient selected from the group consisting of: vitamin E, Berberine (Berberis vulgaris extract), lycopene, Coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean; and
e) one or more ingredients identical to or different from one another selected from: a filler, a thickener, a preservative, a stabiliser, an acidulant, a sweetener and a flavouring; and
f) optionally, water until reaching 100 % by weight of the composition.

In another embodiment of this aspect of the invention, optionally combined with any other embodiment of the invention described herein above or below, the method comprises the mixture of a) phytosterols and/or phytostanols, or an extract comprising phytosterols and/or phytostanols; b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof; c) Hydroxytyrosol, or an extract comprising Hydroxytyrosol; and d) at least one ingredient selected from the group consisting of: vitamin E, Berberine (Berberis vulgaris extract), lycopene, coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean; and
e) one or more ingredients identical to or different from one another selected from: a filler, a thickener, a preservative, a stabiliser, an acidulant, a sweetener and a flavouring; and
f) water until reaching 100 % by weight of the composition.

In a preferred embodiment of this aspect of the invention, optionally combined with any other embodiment of the invention described herein above or below, the at least one agent capable of treating dyslipidemia in the method is selected from Monacolin K, or an extract comprising Monacolin K.

In one embodiment of the invention, optionally combined with any other embodiment of the invention described herein above or below, the order of the compounds to be mixed is as follows (by way of example, the agent capable of treating dyslipidemia has been selected herein from Monacolin K or an extract comprising Monacolin K): (i) mixing the water with the thickener or thickeners; (ii) adding the filler or fillers; (iii) adding the mixture of phytosterols and/or phytostanols or the extract containing them; (iv) adding Monacolin K or the extract comprising Monacolin K; (v) adding the preservative or preservatives; (vi) adding the sweetener or sweeteners; (vii) adding the stabiliser or stabilisers; (vii) adding sodium chloride; (viii) adding the acidulant or acidulants; (ix) optionally adding vitamin E; (x) adding Hydroxytyrosol. After adding each of the compounds of (i) to (x), stir between 5 and 30 minutes. Thus, in another embodiment of the invention, optionally combined with any other embodiment of the invention described herein above or below, the mixture is carried out by stirring.

Preferably, the preparation of the composition is carried out at a temperature between 20 and 60 °C, preferably between 20 and 50 °C, more preferably between 20 and 40 °C, and even more preferably between 20 and 30 °C. Thus, in one embodiment of the invention, optionally combined with any other embodiment of the invention described herein above or below, the mixture is carried out at a temperature between 20 and 30 °C, or more preferably between 22 and 28 °C. In another embodiment, the temperature is 25 °C.

As shown in Example 3, the composition of the invention obtained from the mentioned method of preparation has a pH of 4.3. Thus, in another embodiment of the invention, optionally combined with any other embodiment of the invention described herein above or below, the pH of the composition is comprised between 3.0 and 5.0. In another embodiment, the pH is comprised between 3.5 and 4.5. Still in another embodiment, the pH of the composition is 3.0, 3.5, 4.0, 4.5 or 5.0.

The expression "functional food", as it is used herein, refers to any healthy or functional food which helps to maintain bodily functions beyond just the basic role of supplying nutrients.

The expression "functional beverage" refers to beverages that have been improved with added ingredients which help to maintain bodily functions beyond just basic nutrition.

The term "therapeutically effective amount" or "therapeutic dose" as it is used herein means the amount of a particular agent that is sufficient to provide a therapeutic benefit in the treatment or prevention of a disease, or in the modulation of the serum lipid and lipoprotein level, or the reduction of blood cholesterol levels.

In one embodiment, optionally combined with any other embodiment of the invention described herein above or below, the composition of the present invention is part of a functional food. It can be used as a food or beverage additive to produce a functional food or a functional beverage. Therefore, it can be added to semisolid products, solid products or liquid products or their derivatives, such as concentrates or powders. Examples of food products are selected from the list consisting of water; milk and derivatives such as yogurts or cheese; beverages including juices, soft drinks, sports drinks or alcoholic beverages; confectionery products such as chocolates, candies or gelatins; pasta; cereals and pastries. However, it can also be formulated as tablets and other forms of pills.

In one embodiment, optionally combined with any other embodiment of the invention described herein above or below, the functional food is selected from the group consisting of beer, alcohol-free beer, tea, milk, pasta, cakes, biscuits, cereal bars, breakfast cereals, puffed grains, bread, crepes, pies, creams and desserts.

The expressions "dietary supplement", "food supplement" or "nutritional supplement", as they are used interchangeably herein, refer to a preparation which seeks to complement the diet and provide nutrients, such as vitamins, minerals, fibres, fatty acids or amino acids, which may be absent or may not be consumed in a sufficient amount in a person's diet.

In another embodiment, optionally combined with any other embodiment of the invention described herein above or below, the composition of the invention is in the form of a dietary supplement. The dietary supplement comprises an effective amount of the composition as defined above together with one or more edible excipients or vehicles.

In one embodiment, optionally combined with any other embodiment of the invention described herein above or below, the composition of the present invention is in the form of a pharmaceutical composition. The pharmaceutical composition comprises a therapeutically effective amount of the composition, as defined above, together with one or more pharmaceutically acceptable excipients or vehicles. The excipients or vehicles which are used are for oral administration, including but not limited to, fillers, binders, disintegrants, lubricants, anti-caking agents, glidants or mixtures thereof.

The pharmaceutical compositions and dietary supplements of the invention can be formulated in numerous forms which include, but are not limited to, solutions, tablets, capsules, granules, suspensions, dispersions, powders, sugar coated tablets, chewable candies, candy bars, concentrates, drops, elixirs, emulsions, films, gels, granules, gums, gelatins, oils, pastas, pills, agglomerates, syrups, chewable gelatin forms, or chewable tablets.

Methods for preparing functional foods, dietary supplements and/or pharmaceutical compositions are well known in the state of the art. Those skilled in the art can readily determine the suitable excipients and/or vehicles and their amounts according to the type of formulation that is being prepared.

The composition according to the invention has an effect of lowering and maintaining cholesterol levels in individuals suffering hypercholesterolemia. The term "hypercholesterolemia" refers to the presence of high blood cholesterol levels. In particular, the composition according to the invention demonstrates very good results in patients with low or moderate cardiovascular risk and/or with low or moderate hypercholesterolemia. These patients are characterised by having a concentration of plasma cholesterol of ≥ 200 mg/dl and/or a concentration of LDL ("*low density lipoproteins*") cholesterol of ≥ 115 mg/dl.

Therefore, the composition of the invention for use in lowering blood cholesterol levels of an individual is part of the invention. It has been verified that after one month taking the product according to the invention, in particular where the agent capable of treating dyslipidemia is selected from Monacolin K or an extract comprising Monacolin K, once a day, as described in Example 4, cholesterol levels are reduced by about 15 %. Therefore, in a three-month treatment, by taking the product once a day with lunch or dinner, right before or during, cholesterol levels can be reduced by 15 to 30 %, generally by about 15 to 25 %. In some patients, such reductions were observed after three months, even more generally the result of the reduction was by about 15 to 20 %.

Besides total plasma cholesterol, in the study according to Example 4 other clinical parameters relevant for cardiovascular risk were measured, which parameters are LDL cholesterol level, apolipoprotein B level, and non-HDL ("*high density lipoprotein";* non-HDL cholesterol is calculated by subtracting the amount of the HDL cholesterol from total cholesterol) cholesterol level. According to the results of Example 4, LDL cholesterol levels dropped by about 20 % after one month taking the composition of the invention and were maintained after 3 months taking the composition of the invention. In the control group a reduction of only about 7 % was observed after three months. Apolipoprotein B levels dropped by about 12 % after one month taking the composition of the invention and up to about 14 % after 3 months taking the composition of the invention. In the control group a reduction of only about 3 % was observed after 3 months. Additionally, non-HDL cholesterol levels dropped by about 16 % after one month taking the composition of the invention and up to about 20 % after 3 months taking the composition of the invention. In the control group a reduction of only about 3 % was observed after 3 months.

Additionally, it was observed that the concentration of the C-reactive protein, a relevant clinical parameter for detecting inflammations, dropped significantly by taking the composition of the invention, whereas in the control group no changes were observed.

This aspect can also be formulated as the use of the composition of the invention as defined above for the preparation of a medicinal product, or a dietary supplement, or a functional food for lowering blood cholesterol levels of an individual.

It can also be formulated as a method for lowering blood cholesterol levels of an individual which comprises administering a therapeutically effective amount of the composition of the invention as defined above, either together with pharmaceutically acceptable excipients or carriers, or in the form of a dietary supplement or functional food to a patient in need of same.

As discussed above, there are different factors which can impart a higher risk of suffering cardiovascular problems, including same age, tobacco abuse, alcohol consumption, dyslipidemia, high blood pressure, diabetes and family history of cardiovascular disease. Thus, in one embodiment, optionally combined with any other embodiment of the invention described herein above or below, the individual to be treated with the composition is one who has a high risk of suffering cardiovascular problems.

In another embodiment, optionally combined with any other embodiment of the invention described herein above or below, the individual suffers from a disease selected from the group consisting of atherosclerosis, familial hypercholesterolemia, diabetes; liver diseases, endocrine diseases and kidney diseases.

The word "comprises" and variations thereof are not intended to exclude other technical features, additives, components or steps throughout the description and claims. For those skilled in the art, after examining the description, the objectives, advantages and additional features of the invention will become apparent or can be learned by putting the invention into practice. The following examples and drawings are provided by way of illustration and do not seek to limit the present invention. The reference signs relating to the drawings and placed in parentheses in a claim only attempt to increase comprehension of the claim and must not be interpreted as limiting the scope of the claim. Furthermore, the present invention covers all the possible combinations of particular and preferred embodiments described herein.

### EXAMPLES

### Reagents:

Methocel E15: hydroxypropylmethyl cellulose
Methocel A15: ether methyl cellulose
Vivapur MCG: A mixture of between 82 and 88 % by weight of microcrystalline cellulose and 12 to 18 % by weight of xanthan.
Tastegem: A mixture of flavourings, tapioca Maltodextrin, corn Maltodextrin, and modified corn Starch (E 1450).
Sucragel: A mixture of glycerin, capric/caprylic triglyceride, water and sucrose laurate.

### Example 1:

60 g of purified water were mixed at room temperature (around 20-25 °C) with thickener Methocel E15, 3 g, and it was then stirred for 30 minutes. Then 1 g of a filler, Vivapur MCG 150, was added and the obtained mixture was stirred for an additional 30 minutes. Next, 4.00 g of 95 % phytosterols were added and it was stirred for 30 more minutes. Then 1.67 g of 1.5 % red yeast rice were added and it was stirred for 15 minutes. Next, 0.3 g of the preservative potassium sorbate were added and it was stirred for 5 minutes. Then the sweetener, 0.024 g of sucralose, was added stirring for 15 minutes. Then 0.27 g of acidulant acid anhydride was added and it was stirred for another 5 minutes. Next, 20 g of the emulsifier Sucragel CF and 6.25 g of 20 % hydroxytyrosol W8 were added. Lastly, 0.1 g of antifoaming agent dimethicone dissolved in 10 ml of water was added and the amount of water needed to reach a final volume of 100 ml was mixed in. To provide taste, 0.125 g of raspberry flavour and 0.5 g of strawberry flavour were added.

The resulting formulation is a stable formulation.

### Example 2:

Example 1 was repeated, with the difference being that 0.20 g of xanthan gum was used instead of Methocel E15, and 16 g of a 25 % emulsion of phytosterols and phytostanols was applied.

The resulting formulation is a stable formulation.

### Example 3:

At room temperature (around 20-25 °C), 800 ml of purified water was mixed with the thickeners glycerine, 100 g, and Methocel A15, 24 g, and it was then stirred for 30 minutes. Then 8 g of a filler, Vivapur MCG 150, was added and the obtained mixture was stirred for an additional 30 minutes. Next, 126.32 g of 95 % phytosterols were added and it was stirred for 30 more minutes. Then 16 g of 5 % red yeast rice were added and it was stirred for 15 minutes. Next, 3 g of the preservative potassium sorbate is added, and it was stirred for 5 minutes. Then sweeteners were added, first 0.24 g of sucralose stirring for 15 minutes, and then 0.2 g of stevia stirring for 10 more minutes. Next, 0.136 g of stabiliser, Tastegem, was added and it was stirred for another 10 minutes. Then 5 g of sodium chloride was added, and it was stirred for 5 minutes. Then 2.4 g of acidulant acid anhydride was added and it was stirred for another 5 minutes. Next, 1.92 g of 50 % water dispersible vitamin E was added and it was stirred for 5 more minutes. Lastly, 2 g of 20 % hydroxytyrosol W8 was added and it was stirred for 5 more minutes. To provide taste, 0.5 g of raspberry flavour and 1.8 g of strawberry flavour were added.

The resulting formulation is a stable formulation with a more pleasing taste than those exemplified in Examples 1 and 2.

The composition obtained in Example 3 had a pH of 4.3 and a liquid refractive index of 11.6 °B.

### Example 4: Clinical trial

A clinical trial is performed as described below to confirm the high efficacy of the composition according to the invention in the control of blood cholesterol levels.

### Objective

### Primary Objective

1. Analyse the change in lipid profile (total cholesterol, LDL cholesterol, HDL cholesterol, non-HDL cholesterol and triglycerides) in patients with hypercholesterolemia after intervention with the composition of the invention in which dietary intake and physical activity have been monitored.
2. Analyse the change after intervention of the oxidised main LDLs (more atherogenic forms of LDL) responsible for the formation of atherosclerotic plaque, after intervention with the composition of the invention.

### Secondary Objectives:

1. Evaluate the change in cardiovascular risk scale determined by REGICOR, after intervention with the composition of the invention.
2. Analyse the change in the main systemic inflammation-related biomarkers the C-reactive protein and the interleukin-6 after intervention with the composition of the invention.

The higher efficacy of the composition of the invention is tested in the following clinical trial. The metabolic effect of the composition of the invention in subjects with hypercholesterolemia, defined by the presence of total cholesterol values > 200 mg/dl - <250 mg/dl without lipid-lowering treatment or prior cardiovascular disease, is evaluated in said study.

Once the selected patients were included in the study, an initial visit is conducted where a complete medical history is provided, diet and exercise recommendations are given, and an appointment was scheduled for the patients after 15 days of stabilisation of hygienic-dietary measures for visit 0. In this visit, the participants had to be fasting (for at least 10 hours) for the baseline blood draw and will initiate intervention of the study with the daily dose of the composition of the invention or a placebo for 12 weeks. During this period, the participants must go to the centre at 4, 8 and 12 weeks. In each of these visits, a clinical examination is performed, dietary intake and physical activity surveys are given, and biochemical parameters are determined.

### 2. Product information

The composition according to the invention in the form of stick liquid is tested in the clinical study as a dietary supplement, which comprises as its main ingredients: 10 mg of Monacolin K
1.5 g of phytosterols
5 mg of olive hydroxytyrosol

This supplement must be taken once a day, preferably right before or at lunch or dinner.

### 3. Inclusion/Exclusion Criteria:

### 3.1 Inclusion Criteria:

- Patients between 35-75 years of age with a low (< 5 %) or moderate (5-9 %) coronary risk according to the REGICOR risk tables (https://www.imim.cat/ofertadeserveis/software-pubiic/regicor/?1)
- Patients with total cholesterol values between 200-250 mg/dl in previous blood work (maximum 3 months) and LDL cholesterol > 115 mg/dl without lipid-lowering treatment.

### 3.2 Exclusion Criteria:

- Patients who are following or have received lipid-lowering treatment (statins, fibrates, resins, ezetimibe) and/or any other product with lipid-lowering effect (red rice, plant sterols) in the last 3 months.
- Patients with prior cardiovascular disease defined by coronary disease in the form of myocardial infarction, angina pectoris, stroke, peripheral arterial disease.
- Patients indicated for treatment with lipid-lowering drugs.
- Patients with a high (10-14 %) or very high (≥15 %) coronary risk according to the REGICOR risk tables
- Patients with BMI ≥ 30

### 4. Tests to be conducted:

4.1. Basic medical history: Earlier medical history information in relation to cardiovascular risk factors (age, tobacco abuse, alcohol consumption, dyslipidemia, blood pressure, diabetes and family history of cardiovascular disease), pharmacological treatment at the start of the study, as well as a physical exam including anthropometric parameters (height, weight and waist circumference) at the start and in each of the visits to the centre.
4.2. Taking blood pressure at the start and in each of the visits to the centre.
   - Leave the patient sitting for 5 minutes in a peaceful room before taking BP measurements.
   - Take 3 clinical BP determinations spaced 1 min apart while seated with a semiautomatic OMRON 705 model apparatus.
   - Use a standard cuff (12-13 cm wide and 35 cm long) but have wider or narrower cuffs on hand for thicker or thinner arms, respectively. Measure BP while standing after 5 minutes of rest in this position. HTA (Arterial hypertension) will be defined as those BP values ≥ 140/90 mmHg.
4.3 Blood work: Data in relation to glycemia, glycated haemoglobin, kidney function (creatinine and glomerular filtrate and microalbuminuria in urine collected in the morning), lipid parameters (total chol., cHDL, cLDL, triglycerides, vLDL, ApoA1, ApoB and lipoprotein a), liver biology (GOT, GPT, GGT, FA, Bi), creatine kinase, LDH and aldolase, is collected at the start and in each of the visits to the centre. Likewise, 20 ml of additional blood will be drawn for the determination of the oxidised LDL particles by OxiSelect™ Human Oxidised LDL ELISA Kits and systemic inflammation-related biomarkers such as ultrasensitive C-reactive protein and interleukin-6.

The present study seeks to prove the beneficial and synergistic effect of the composition of the invention as a first step in the treatment of mild primary hypercholesterolemia in patients without any medical indication of specific treatment with statins or other lipid-lowering drugs, but with high cholesterol levels.

### 5. Result

A total of 40 patients participated in the clinical trial in which 20 patients took the product according to point 2. (product according to the invention) and the other 20 patients took a placebo as a control group. The patients were on average 60 years old, 60 % of them were women, and they had similar anthropometric characteristics and overall cardiovascular risk global without any significant differences. Average cholesterol levels before the study began were around 236 mg/dl in the case of the group that took the product and around 232 mg/dl in the case of the control group.

After a month of treatment with the product, a significant reduction of total cholesterol values was observed (-29.00 ± 17 mg/dl product group vs. -1.2 ± 10.37 mg/dl control group), LDL cholesterol (-30.83 ± 18.49 mg/dl product group vs. -6.0 ± 16.61 mg/dl control group) and non-HDL (-28.66 ± 15.45 mg/dl product group vs. -3.28 ± 13.88 mg/dl control group). That is, taking the product for a month reduces cholesterol levels by 15 %. The reduction of cholesterol levels is expected to even further increase, up to 20 or 25 %, after following treatment for 3 months.

Besides total plasma cholesterol, other relevant clinical parameters were measured for cardiovascular risk, which parameters are LDL cholesterol level, apolipoprotein B level, and non-HDL ("*high density lipoprotein";* non-HDL cholesterol is calculated by subtracting the amount of the HDL cholesterol from total cholesterol) cholesterol level. It was observed that LDL cholesterol levels dropped by about 20 % after one month taking the composition of the invention and were maintained after 3 months taking the composition of the invention. In the control group a reduction of only about 7 % was observed after three months. Apolipoprotein B levels dropped by about 12 % after one month taking the composition of the invention and up to about 14 % after 3 months taking the composition of the invention. In the control group a reduction of only about 3 % was observed after 3 months. Non-HDL cholesterol levels dropped by about 16 % after one month taking the composition of the invention and up to about 20 % after 3 months taking the composition of the invention. In the control group a reduction of only about 3 % was observed after 3 months.

Additionally, it was observed that the concentration of the C-reactive protein, a relevant clinical parameter for detecting inflammations, dropped significantly by taking the composition of the invention, whereas in the control group no changes were observed.

### CITED LITERATURE

- Capasso R, Evidente A, Avolio S., Solla F, "A high convenient synthesis of hydroxytyrosol and its recovery from agricultural waste waters", J. Agr. Food Chem. 1999, 47; 1745-1748
- Fleming H P, Walter W M, Etchells J L, "Antimicrobial properties of Oleuropein and products of its hydrolysis from green olives" App. Microbio 1973; 26: 777-782
- Yuan JJ, Wang CZ, Ye JZ, Tao R, Zhang YS, "Enzymatic hydrolysis of oleuropein from Olea eropea (olive) leaf extract and antioxidant activities" Molecules 2015, 20(2):2903-21).

## Claims

1. A composition comprising:
a) phytosterols and/or phytostanols, or an extract comprising phytosterols and/or phytostanols;
b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof;
c) hydroxytyrosol, or an extract comprising hydroxytyrosol.

2. The composition according to claim 1 comprising:
a) phytosterols and/or phytostanols, or an extract comprising phytosterols and/or phytostanols;
b) Monacolin K, or an extract comprising Monacolin K; and
c) hydroxytyrosol, or an extract comprising hydroxytyrosol.

3. The composition according to any of claims 1 to 2, wherein the amount for daily administration with respect to the composition of phytosterols and/or phytostanols is comprised between 0.1 g and 10 g, the amount for daily administration with respect to the composition of Monacolin K is comprised between 0.1 mg and 20 mg, and the amount for daily administration with respect to the composition of hydroxytyrosol is comprised between 0.1 mg and 20 mg, wherein the mentioned amounts of active ingredient are with respect to the total grams of the composition.

4. The composition according to any of claims 1 to 3, wherein the % by weight of phytosterols and/or phytostanols in the extract comprising phytosterols and/or phytostanols is comprised between 1 % and 100 % by weight of the extract; wherein the extract comprising Monacolin K is a red yeast rice extract and the % by weight of Monacolin K in the red yeast rice extract is comprised between 0.001 % and 100 % by weight of the extract; and, wherein the % by weight of hydroxytyrosol in the extract which comprises it is comprised between 1 % and 100 % by weight of the extract.

5. The composition according to any of claims 1 to 4, wherein the % by weight with respect to the composition of phytosterols and/or phytostanols is comprised between 5 and 15 %, the % by weight with respect to the composition of Monacolin K is comprised between 0.03 and 0.15 %, and the % by weight with respect to the composition of hydroxytyrosol is comprised between 0.01 and 0.08 %.

6. The composition according to any of claims 1 to 5, wherein the % by weight with respect to the composition of the extract comprising phytosterols and/or phytostanols is comprised between 5 and 30 %, the % by weight with respect to the red yeast rice extract is comprised between 0.5 and 5 %, and the % by weight with respect to the composition of the extract comprising Hydroxytyrosol is comprised between 0.02 and 8 %.

7. The composition according to any of claims 1 to 6, further comprising at least one ingredient selected from the group consisting of: vitamin E, Berberine (Berberis vulgaris extract), lycopene, Coenzyme Q10, Ubiquinol, artichoke (Cynara scolymus extract), desmodium (Desmodium adscendens extract), black radish (Raphanus sativus nigra extract), milk thistle (Silybum marianum extract), policosanols, beta-glucans (for example from barley or oat) and carob bean.

8. The composition according to claim 7, wherein the % by weight with respect to the composition of vitamin E is comprised between 0.01 and 5 %; or wherein the amount for daily administration with respect to the composition of vitamin E is comprised between 1 and 500 mg.

9. The composition according to any of claims 1 to 8, further comprising one or more ingredients identical to or different from one another selected from: a filler, a thickener, a preservative, a stabiliser, an acidulant, a sweetener and a flavouring; optionally the composition is in solid, semi-solid, gel or liquid form.

10. The composition according to claim 9, which is in liquid form and comprises a liquid vehicle which is selected from the group comprising water, vegetable oil, milk or ethanol.

11. A method for preparing the composition as defined in any of claims 1 to 10, which method comprises mixing the following components:
a) phytosterols and/or phytostanols, or an extract comprising phytosterols and/or phytostanols;
b) at least one agent capable of treating dyslipidemia selected from the group consisting of Monacolin K, an extract comprising Monacolin K, policosanol, a sugarcane wax extract comprising policosanol, Annur apple (Melannurca campana) extract, berberine, an extract of plants from the Berberis family (such as, for example, Berberis aristata, Berberis vulgaris, Berberis aquifolium), bergamot (Citrus bergamia), Garlic (Alium sativum) extract, Coriander or Cilantro (Coriandrum sativum) extract, green tea (Camelia sinensis) extract, pantethine, pantothenic acid; and mixtures thereof;
c) Hydroxytyrosol, or an extract comprising Hydroxytyrosol; and
d) optionally, at least one ingredient selected from the group consisting of: vitamin E, berberine (*Berberis vulgaris* extract), lycopene, coenzyme Q10, Ubiquinol, artichoke (*Cynara scolymus* extract), desmodium (*Desmodium adscendens* extract), black radish (*Raphanus sativus nigra* extract), milk thistle (*Silybum marianum* extract), policosanols, beta-glucans (for example from barley or oat) and carob bean; and
e) optionally, one or more ingredients identical to or different from one another selected from: a filler, a thickener, a preservative, a stabiliser, an acidulant, a sweetener and a flavouring; and
f) optionally, a liquid vehicle until reaching 100 % by weight of the composition.

12. A food, comprising a therapeutically effective amount of the composition as defined in any of claims 1 to 10, together with one or more edible excipients or vehicles.

13. A pharmaceutical composition comprising a therapeutically effective amount of the composition as defined in any of claims 1 to 10, together with pharmaceutically acceptable excipients or carriers.

14. A composition as defined in any of claims 1 to 10, for use in controlling and reducing blood cholesterol levels of an individual.

15. The composition for use according to claim 14, wherein the individual suffers a disease selected from the group consisting of atherosclerosis, familial hypercholesterolemia, diabetes, liver diseases, endocrine diseases and kidney diseases.
